(19) Europäisches Patentamt
European Patent Office
Office européen des brevets

(11) Publication number: **0 059 258**
**B1**

(12) # EUROPEAN PATENT SPECIFICATION

(45) Date of publication of patent specification: **30.05.84**

(21) Application number: **81109393.9**

(22) Date of filing: **30.10.81**

(51) Int. Cl.$^3$: **C 07 C 57/03**, C 07 C 69/533, C 07 C 69/587, C 07 C 103/133, C 07 C 49/203, A 61 K 31/12, A 61 K 31/23, A 61 K 31/16

(54) **Pharmaceutical preparations comprising polyprenyl compounds, especially as anti-cancer agents, and pharmaceutical compositions for the prevention and treatment of cancer and skin diseases.**

(30) Priority: **24.12.80 JP 182116/80**
**30.04.81 JP 64244/81**

(43) Date of publication of application:
**08.09.82 Bulletin 82/36**

(45) Publication of the grant of the patent:
**30.05.84 Bulletin 84/22**

(84) Designated Contracting States:
**BE CH DE FR GB IT LI NL SE**

(56) References cited:
**EP-A-0 041 235**
**FR-A-2 479 807**

(73) Proprietor: **Eisai Co., Ltd.**
**6-10, Koishikawa 4-chome Bunkyo-ku**
**Tokyo 112 (JP)**

(72) Inventor: **Yamatsu, Isao**
**1-14-29-509, Kamiaoki-cho**
**Kawaguchi-shi Saitama (JP)**
Inventor: **Inai, Yuichi**
**Tokyu Hasune Haitsu, 3-11-25-702**
**Hasune, Itabashi Tokyo (JP)**
Inventor: **Abe, Shinya**
**3-2-5, Nakamura Nerima-ku**
**Tokyo (JP)**
Inventor: **Suzuki, Takeshi**
**144-10, Wakamatsu**
**Abiko-shi Chiba (JP)**
Inventor: **Suzuki, Yoshikazu**
**6, Aza Sakuranosato Kouda, Asai-cho**
**Ichinomiya-shi Aichi (JP)**
Inventor: **Tagaya, Osamu**
**1648-13, Kano**
**Gifu-shi Gifu (JP)**
Inventor: **Suzuki, Kouichi**
**1-59, Ozakikitamachi**
**Kakamigahara-shi Gifu (JP)**
Inventor: **Abe, Kouichi**
**3-52-7, Yotsuya**
**Fuchu-shi Tokyo (JP)**

**EP 0 059 258 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Yamada, Kouji**
**1-21-17, Tokiwadai**
**Itabashi-ku Tokyo (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**Description**

This invention relates to pharmaceutical preparations comprising a polyprenyl compound having the formula (I):

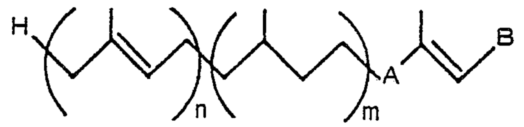 (I)

in which each of *n* and *m* is 0, 1 or 2, n + m is 0, 1 or 2, A is

A is 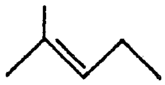 , 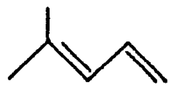 , or 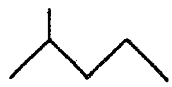 , and B is

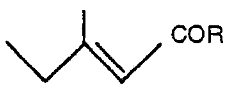 , 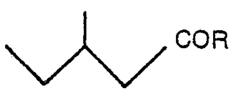 , 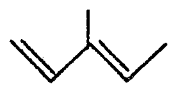 , 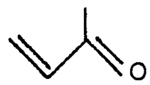 ,

or 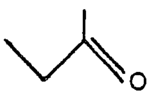

[in which R represents the hydroxyl group, a lower alkoxy group, or

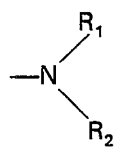

(wherein each of $R_1$ and $R_2$ is the hydrogen atom, a lower alkyl group or an aryl group)]; provided that R is a lower alkoxy or

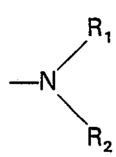 ,

if *n* is 1, *m* is 0, A is

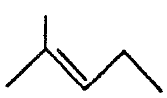 , and B is 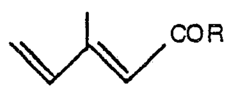

especially as anti-cancer agent, and method of application of them for the prevention and treatment of cancer and skin diseases.

Examples of the lower alkoxy groups represented by R in the above-mentioned formula (I) include methoxy, ethoxy, i-propoxy, n-propoxy, t-butoxy and n-butoxy. Examples of the lower alkyl groups represented by $R_1$ and $R_2$ include methyl, ethyl, i-propyl, n-propyl, t-butyl and n-butyl, and examples of the aryl groups represented by $R_1$ and $R_2$ include phenyl and a phenyl group having substituent groups such as hydroxyl, a lower alkyl group or halogen. If R in the formula (I) is hydroxyl, the compound may be in the form of a salt such as sodium or potassium salt.

W. Bollag et al. reported in Europ. J. Cancer, Vol. 10, p 731 (1974) that retinoids such as ethyl 9-(2,3,6-trimethyl-4-methoxyphenyl)-3,7-dimethyl-2,4,6,8-nonatetraenoate have anti-cancer activity. These retinoide compounds, however, are highly toxic, and further have problems such as causing hypervitaminosis of Vitamin A when administered.

The present invention provides an anti-cancer agent comprising the polyprenyl compound of the

formula (I). The polyprenyl compounds of the formula (I) show remarkable anti-cancer activity and are highly safe compounds. For instance, these polyprenyl compounds do not cause hypervitaminosis of Vitamin A. Further, toxicities of the polyprenyl compounds of the formula (I) other than the hypervitaminosis are also at low level.

Moreover, the polyprenyl compounds of the formula (I) are of value as therapeutic agents for treatment of skin diseases with keratinization or treatment of allergic or inflammatory skin diseases, such as psoniasis, acne, acne vulgaris, Darier's disease, palmoplantar pustulosis, lichen plusnus, ichthyosis, erythroderma, pityriasis rubra pilasis, and keratosis senilis, as well as therapeutic agents for prevention and treatment of cancer and precancerous conditions.

Some of the polyprenyl compounds of the formula (I) are already known. For instance, some polyprenyl compounds represented by the formula (I) are described in Japanese Patent Provisional Publication 54—5042, Helv. Chim. Acta., Vol. 35, p. 1649 (1952), Agr. Biol. Chem., Vol. 34, No. 8, p. 1184 (1970), and J. Chem. Soc. (C) p. 2154 (1966). However, these references do not describe the anti-cancer activity of these compounds.

Examples of the known polyprenyl compounds represented by the formula (I) include:

6,10,14-trimethyl-3,5,9,13-pentadecatetraen-2-on
6,10,14-trimethyl-3,5-pentadecadien-2-on
6,10,14,18-tetramethyl-3,5,9,13,17-nonadecapentaen-2-on
6,10,14,18-tetramethyl-3,5,9,17-nonadecatetraen-2-on
6,10,14,18-tetramethyl-5,9,13,17-nonadecatetraen-2-on
6,10,14,18-tetramethyl-3,5-nonadecadien-2-on
3,7,11,15-tetramethyl-2,6,10,14-hexadecatetraenoic acid
ethyl 3,7,11,15-tetramethyl-2,6,10,14-hexadecatetraenate
3,7,11-trimethyl-2,4,6,10-dodecatetraenoic acid
ethyl 3,7,11-trimethyl-2,4,6,10-dodecatetraenoate
methyl 3,7,11,15-tetramethyl-2,4,6,10,14-hexadecahexaenoate.

Examples of the novel polyprenyl compounds represented by the formula (I) include:

3,7,11,15,19-pentamethyl-2,4,6,10,14,18-eicosahexaenoic acid
3,7,11,15-tetramethyl-2,4,6,14-hexadecatetraenoic acid
3,7,11,15-tetramethyl-2,4,6-hexadecatrienoic acid
3,7,11,15-tetramethyl-2,4,6,8,10,14-hexadecahexaenoic acid
3,7,11,15-tetramethyl-2,4,6,10-hexadecatetraenoic acid
3,7,11,15,19-pentamethyl-2,4,6,8,10,14,18-eicosaheptaenoic acid
3,7,11,15,19-pentamethyl-2,4,6,10,18-eicosapentaenoic acid
ethyl 3,7,11,15-tetramethyl-2,4,6-hexadecatrienoate
ethyl 3,7,11,15,19-pentamethyl-2,4,6,10,14,18-eicosahexaenoate
methyl 3,7,11,15,19-pentamethyl-2,4,6,10,14,18-eicosahexaenoate
ethyl 3,7,11,15-tetramethyl-2,4,6,8,10,14-hexadecahexaenoate
3,7,11,15-tetramethyl-2,4,6,10,14-hexadecapentaenoamide
N-(p-hydroxyphenyl)-3,7,11,15-tetramethyl-2,4,6,10,14-hexadecapentaenoamide
N-ethyl-3,7,11,15-tetramethyl-2,4,6,10,14-hexadecapentaenoamide
N,N-dimethyl-3,7,11,15-tetramethyl-2,4,6,10,14-hexadecapentaenoamide
N-ethyl-3,7,11,15-tetramethyl-2,4,6,8,10,14-hexadecahexaenoamide
N-ethyl-3,7,11,15,19-pentamethyl-2,4,6,10,14,18-eicosahexaenoamide
ethyl 3,7,11,15-tetramethyl-2,4,6,10,14-hexadecapentaenoate
propyl 3,7,11,15-tetramethyl-2,4,6,10,14-hexadecapentaenoate
3,7,11,15-tetramethyl-6,10,14-hexadecapentaenoic acid
N,N-dimethyl-3,7,11,15-tetramethyl-6,10,14-hexadecatrienoamide
N-ethyl-3,7,11,15-tetramethyl-2,6,10,14-hexadecatrienoamide.

The novel polyprenyl compounds represented by the formula (I) such as exemplified above can be prepared in the manner described in the aforementioned publications, in the process given hereinafter, or in a conventional manner.

The results of the pharmacological tests and toxicity tests on the polyprenyl compounds of the formula (I) are set forth below.

### Pharmacological Tests (Anti-cancer Activity)

(1) Test Procedure

A mouse (ICR strain, female, 60 days age) was shaved at the back of neck (to the extent of 5 $cm^2$). 7,12-Dimethylbenzo-[2]-anthracene was dissolved in acetone to give 75 mg/100 ml. solution. The so prepared solution was applied to the mouse on the 60th day of age and further on the 75th day of age in the amount of 0.2 ml per mouse.

Croton oil was dissolved in acetone to give 250 mg/100 ml solution, and the so prepared solution was applied to the mouse in the amount of 0.2 ml per mouse, twice a week until the treatment was started. When 3—7 papillomata (diameter of 3—8 mm for each, and total diameter of 30—60 mm) were produced for a mouse, the treatment was started.

The compound to be tested (test compound) was dissolved in groundnut oil to give 20 mg/ml solution, and administered orally to the mouse. The solution was administered 10 times for 14 days (once a day), and the diameters of the papillomata were measured on the 14th day to determine the total diameter for each mouse. Ratio of increase or decrease of the papillomata was calculated from the total diameter on the 14th day and the total diameter measured prior to the starting of administration of the test compound. This value was adopted for evaluating the anti-cancer activity.

(2) Results

TABLE 1

| Test Compound | Number of mouse | Ratio of increase or decrease of papillomas (%) |
|---|---|---|
| Groundnut only (Control) | 3 | + 17.1 |
| (1) 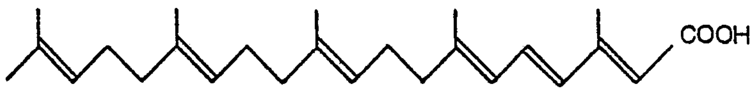 | 5 | - 24.0 |
| (2) 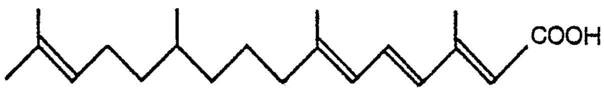 | 4 | - 19.5 |
| (3) 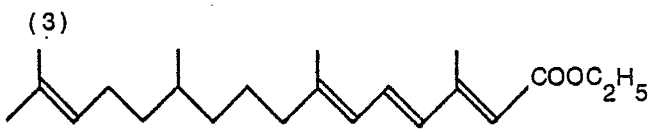 | 4 | - 10.2 |
| (4) 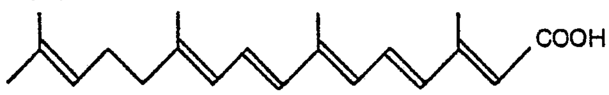 | 5 | - 26.3 |
| (5) 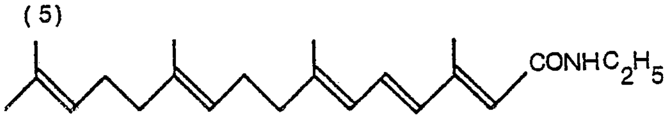 | 5 | - 25.5 |
| (6) 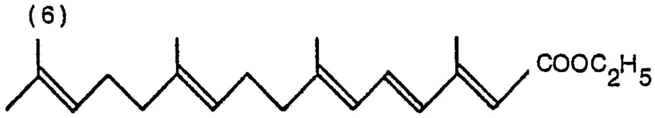 | 17 | - 17.5 |
| (7) 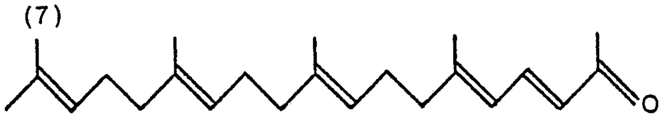 | 5 | - 12.7 |
| (8) 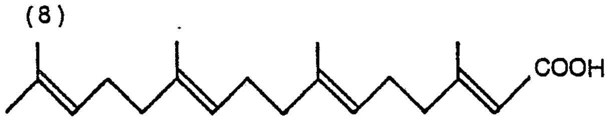 | 7 | - 6.5 |
| (9) 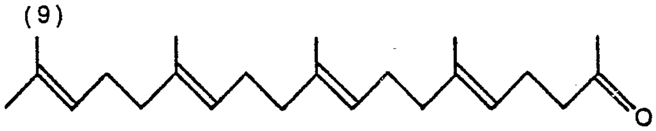 | 7 | - 8.2 |

Remarks: The compounds identified by the structural formulae in Table 1 correspond to the following polyprenyl compounds.

(1) 3,7,11,15,19-pentamethyl-2,4,6,10,14,18-eicosahexaenoic acid
(2) 3,7,11,15-tetramethyl-2,4,6,14-hexadecatetraenoic acid
(3) ethyl 3,7,11,15-tetramethyl-2,4,6,14-hexadecatetraenoate
(4) 3,7,11,15-tetramethyl-2,4,6,8,10,14-hexadecahexaenoic acid
(5) 3,7,11,15-tetramethyl-2,4,6,10,14-hexadecapentaenoamide
(6) ethyl 3,7,11,15-tetramethyl-2,4,6,10,14-hexadecapentaenoate
(7) 6,10,14,18-tetramethyl-3,5,9,13,17-nonadecapentaen-2-on
(8) 3,7,11,15-tetramethyl-2,6,10,14-hexadecatetraenoic acid
(9) 6,10,14,18-tetramethyl-5,9,13,17-nonadecatetraen-2-on

As seen from the data in Table 1, the polyprenyl compounds of the formula (I) are effective against the papilloma.

Toxicity Tests

(1) Test procedure

The test compound was administered repeatedly to a group of 6 mice (ICR strain, female) for 14 days in the dosage of 200 mg/kg/day. In the course of the administration procedures, increase or decrease of the weight of the mouse, occurrance of death, etc. were observed.

(2) Test compound

The compounds set forth in the above Table 1 were employed.

(3) Test results

No death was observed. Decrease of the weight was not observed, and a little increase of the weight was observed. No symptoms indicating the effects of side actions, such as falling-out of hair, cyanosis, etc., were observed.

The decrease of the weight and the falling-out of hair are known as indicating the hypervitaminosis of Vitamin A. Accordingly, the results are considered to indicate that the polyprenyl compounds of the formula (I) do not cause the hypervitaminosis of Vitamin A.

In view of the pharmacological test results and the toxicity test results as described hereinbefore, the polyprenyl compounds of the formula (I) are judged to be of high safety and to be of value as anti-cancer agents for prevention and treatment of cancer and precancerous conditions.

It is well known that mouse papillomate as mentioned above are useful as a model for proliferous, allergic keratinization. Accordingly it is understood from the above shown data that the compound according to the invention is effective also against skin diseases with keratinization.

For the application as anti-cancer agent, the polyprenyl compound of the formula (I) can be administered orally in the form of powder, granule, pellet, hard capsule, etc., or parenterally in the form of ointment, suppository, injection solution, etc. The dosage is generally set in the range of 40 mg to 4 g/day for an adult. If the polyprenyl compound of the formula (I) is applied in the form of an external preparation, the dosage can be varied depending on the largeness of area on the affected part. The above-mentioned preparations can be prepared from the polyprenyl compound and generally employable carriers for the medical use by utilizing the conventional methods.

The following examples will illustrate processes for preparing the polyprenyl compounds of the formula (I) and the preparations comprising the polyprenyl compounds, but these examples are not given to restrict the present invention.

Preparation Example 1

*Ethyl 3,7,11,15-tetramethyl-2,4,6,14-hexadecatetraenoate*

To a suspension of 2.5 g of 55% sodium hydride (in oil) in 30 ml of n-hexane was added 13.6 g of triethyl phosphonoacetate. The mixture was then heated under reflux, and 10 g of 6,10,14-trimethyl-3,5,13-pentadecatrien-2-on was added dropwise to the mixture under stirring. After 30 minutes, the reaction liquid was poured into 100 ml of ice-water, and then 200 ml of n-hexane was added for extraction. The n-hexane phase was separated, washed with two 50 ml portions of a mixture of methanol and water (2:1), and concentrated. The so obtained concentrate was purified by the silica gel column chromatography to give 9.0 g of the desired product as an oil.

Analysis for $C_{22}H_{36}O_2$

| | C | H |
|---|---|---|
| Calculated (%) | 79.46 | 10.92 |
| Found (%) | 79.74 | 11.04 |

NMR spectrum (δ, $CDCl_3$): 0.87 (3H, d, J = 6 Hz), 1.28 (3H, t, J = 7Hz), 1.0—1.6 (7H), 1.61 (3H, s), 1.69 (3H, s), 1.85 (3H, s), 1.9—2.4 (4H), 23.4 (3H, d, J = 1Hz), 4.17 (2H, q, J = 7Hz), 5.10 (1H, t, J = 7Hz), 5.75 (1H, bs), 5.95 (1H, d, J = 11Hz), 6.16 (1H, d, J = 15Hz), 6.86 (1H, dd, J = 15Hz, 11Hz)

Mass spectrum (m/e): 332 ($M^+$)

Preparation Example 2

*3,7,11,15-Tetramethyl-2,4,6,14-hexadecatetraenoic acid*

To 8.0 g of the ethyl 3,7,11,15-tetramethyl-2,4,6,14-hexadecatetraenoate obtained in the previous Preparation Example 1 was added to a solution of 3.2 g of potassium hydroxide in 20 ml of isopropyl alcohol, and the mixture was stirred at 50°C for 1 hour. The reaction liquid was then poured into ice-water, made acidic by addition of hydrochloric acid, and extracted with 50 ml of diethyl ether. The ether phase was washed with water, dried over magnesium sulfate, and concentrated to give 7 g of an oil. The oil was dissolved in 40 ml of n-hexane and crystallized at —20°C to give 3.1 g of the desired product as white crystals.

M.p.: 60—62°C

Analysis for $C_{20}H_{32}O_2$

| | C | H |
|---|---|---|
| Calculated (%) | 78.89 | 10.59 |
| Found (%) | 78.77 | 10.63 |

NMR spectrum (δ, $CDCl_3$): 0.87 (3H, d, J = 6Hz), 1.0—1.6 (7H), 1.60 (3H, s), 1.69 (3H, s), 1.85 (3H, s) 1.9—2.3 (4H), 2.34 (3H, d, J = 1Hz), 5.10 (1H, t, J = 7Hz), 5.77 (1H, bs), 5.97 (1H, d, J = 11Hz), 6.20 (1H, d, J = 15Hz), 6.91 (1H, dd, J = 15Hz, 11Hz), 9.6 (1H, b)

Mass spectrum (m/e): 304 ($M^+$)

Preparation Example 3

*3,7,11,15-Tetramethyl-2,4,6,8,10,14-hexadecahexaenoic acid*

To a suspension of 30.3 g of sodium ethoxide in 300 ml of tetrahydrofuran was added 118 g of diethyl 3-ethoxycarbonyl-2-methyl-2-propenylphosphonate. To the mixture was added 67 g of 3,7,11-trimethyl-2,4,6,10-dodecatetraen-1-al under stirring, chilling with ice and shielding from the light. After 1 hour, the reaction liquid was poured into 1 liter of water, and 1 liter of n-hexane was added for extraction. The n-hexane phase was separated, washed with two 100 ml portions of a mixture of methanol and water (2:1), and concentrated to give 99 g of a concentrate. To a refluxing solution of 8.2 g of potassium hydroxide and 80 ml of isopropyl alcohol was added 21 g of the concentrate under shielding from the light. After 15 minutes, the reaction liquid was poured into 300 ml of ice-water, made acidic by addition of hydrochloric acid, and extracted with 300 ml of diethyl ether. The extract was washed with three 100 ml portions of water, dried over magnesium sulfate, and evaporated to remove the solvent. The residue was dissolved in 200 ml of n-hexane and chilled to —20°C to crystallize. There was obtained 9.8 g of the desired product as pale yellow crystals.

Analysis for $C_{20}H_{28}O_2$

| | C | H |
|---|---|---|
| Calculated (%) | 79.95 | 9.39 |
| Found (%) | 80.22 | 9.47 |

NMR spectrum (δ, $CDCl_3$): 1.63 (3H, s), 1.69 (3H, s), 1.84 (3H, s), 1.99 (3H, s), 2.0—2.3 (4H), 2.36 (3H, s), 5.15 (1H, m), 5.6—7.2 (7H, m), 1.04 (1H, b)

Mass spectrum (m/e): 300 ($M^+$)

Preparation Example 4

*3,7,11,15,19-Pentamethyl-2,4,6,10,14,18-eicosahexaenoic acid*

In 100 ml of tetrahydrofuran was dissolved 12 g of 1-p-tolylsulfonyl-3,7,11,15-tetramethyl-2,6,10,14-hexadecatetraene, and the solution was chilled to —50°C. To the solution was added

dropwise 18.5 ml of 15% n-butyllithium — n-hexane solution under stirring and in a stream of nitrogen, maintaining the temperature of the solution at −50°C. Then, 300 ml of tetrahydrofuran solution containing 5.7 g of ethyl 4-bromo-3-methyl-2-butenate was added dropwise to the so produced solution. After 30 minutes, 100 ml of 10% aqueous ammonium chloride solution was added, and then the mixture was allowed to stand to reach room temperature. The mixture was subsequently extracted with two 200 ml portions of n-hexane. The n-hexane phase was washed with three 100 ml portions of water, dried over magnesium sulfate, and evaporated to remove the solvent. There was obtained 14 g of ethyl 3,7,11,15,19-pentamethyl-5-p-tolylsulfonyl-2,6,10,14,18-eicosapentaenoate.

To 4.1 g of potassium hydroxide in 50 ml of isopropyl alcohol was added 12 g of the above-obtained ethyl 3,7,11,15,19-pentamethyl-5-p-tolylsulfonyl-2,6,10,14,18-eicosapentaenoate, and the mixture was stirred at 50°C for 3 hours. The reaction liquid was poured into ice-water, made acidic by addition of hydrochloric acid, and extracted with 100 ml of diethyl ether. The extract was washed with water, dried over magnesium sulfate and evaporated to remove the solvent. There was obtained 8.5 g of an oil. The so obtained oil was dissolved in 40 ml of n-hexane and chilled to −20°C to crystallize. There was obtained 2.3 g of the desired product as white crystals.

M.p.: 45.5—46.5°C

Analysis for $C_{25}H_{38}O_2$

| | C | H |
|---|---|---|
| Calculated (%) | 81.03 | 10.34 |
| Found (%) | 80.89 | 10.52 |

NMR spectrum (δ, $CDCl_3$): 1.60 (9H, s), 1.68 (3H, s), 1.86 (3H, s), 1.9—2.3 (12H), 2.33 (3H, s), 5.09 (3H, b), 5.76 (1H, bs), 5.96 (1H, d, J = 10Hz), 6.18 (1H, d, J = 15Hz), 6.89 (1H, dd, J = 15Hz, 10Hz), 10.2 (1H, b)

Mass spectrum (m/e): 370 ($M^+$)

Preparation Example 5

*Ethyl 3,7,11,15-tetramethyl-2,4,6-hexadecatrienoate*

The procedures described in Preparation Example 1 were repeated using 6,10,14-trimethyl-3,5-pentadecadien-2-on to obtain the desired product as an oil.

Analysis for $C_{22}H_{38}O_2$

| | C | H |
|---|---|---|
| Calculated (%) | 78.98 | 11.45 |
| Found (%) | 79.16 | 11.56 |

NMR spectrum (δ, $CDCl_3$), 0.87 (9H, d, J = 7Hz), 1.27 (3H, t, J = 7Hz), 0.9—1.6 (12H), 1.84 (3H, s), 2.08 (2H, t, J = 7Hz), 2.34 (3H, s), 4.16 (2H, q, J = 7Hz), 5.74 (1H, bs), 5.95 (1H, d, J = 11Hz), 6.16 (1H, d, J = 15Hz), 6.85 (1H, dd, J = 15Hz, 11 Hz)

Mass spectrum (m/e): 334 ($M^+$)

Preparation Example 6

*3,7,11,15-Tetramethyl-2,4,6-hexadecatrienoic acid*

The procedures described in Preparation Example 2 were repeated using the ethyl 3,7,11,15-tetramethyl-2,4,6-hexadecatrienoate obtained in Preparation Example 5 to carry out the hydrolysis. There was obtained the desired product as white crystals.

M.p.: 84.5—85.5°C

Analysis for $C_{20}H_{34}O_2$

| | C | H |
|---|---|---|
| Calculated (%) | 78.38 | 11.18 |
| Found (%) | 78.35 | 11.21 |

NMR spectrum (δ, $CDCl_3$): 0.87 (9H, d, J = 7Hz), 0.9—1.6 (12H), 1.84 (3H, s), 2.09 (2H, t, J = 7Hz), 2.35 (3H, s), 5.76 (1H, bs), 5.96 (1H, d, J = 11Hz), 6.19 (1H, d, J = 15Hz), 6.90 (1H, dd, J = 15Hz, 11Hz), 11.5 (1H, b)

Mass spectrum (m/e): 306 ($M^+$)

## Preparation Example 7

*Ethyl 3,7,11,15-tetramethyl-2,4,6,10,14-hexadecapentaenoate*

To a suspension of 5.0 g of 55% sodium hydride (in oil) in 60 ml of n-hexane was added 28.6 g of triethyl phosphonoacetate. The mixture was then heated under reflux, and 20 g of 6,10,14-trimethyl-3,5,9,13-pentadecatetraen-2-on was added dropwise to the mixture under stirring. After 30 minutes, the reaction liquid was poured into 200 ml of ice-water, and then 500 ml of n-hexane was added for extraction. The n-hexane phase was separated, washed with two 100 ml portions of a mixture of methanol and water (2:1), and concentrated. The so obtained concentrate was purified by silica gel column chromatography to give 18 g of the desired product as an oil.

Analysis for $C_{22}H_{34}O_2$

| | C | H |
|---|---|---|
| Calculated (%) | 79.95 | 10.37 |
| Found (%) | 80.11 | 10.26 |

NMR spectrum (δ, $CDCl_3$): 1.28 (3H, t, J = 7Hz), 1.60 (6H, s), 1.68 (3H, s), 1.85 (3H, s), 1.9—2.3 (8H), 2.33 (3H, d, J = 1Hz), 4.16 (2H, q, J = 7Hz), 5.09 (2H, b), 5.73 (1H, bs), 5.96 (1H, d, J = 11Hz), 6.16 (1H, d, J = 15Hz), 6.85 (1H, dd, J = 15Hz, 11Hz)

Mass spectrum (m/e): 330 ($M^+$)

## Preparation Example 8

*3,7,11,15-Tetramethyl-2,4,6,10,14-hexadecapentaenoamide*

To 3.9 g of potassium hydroxide in 30 ml of isopropyl alcohol was added 10 g of the ethyl 3,7,11,15-tetramethyl-2,4,6,10,14-hexadecapentaenoate obtained in Preparation Example 7, and the mixture was stirred at 50°C for 1 hour. The reaction liquid was poured into ice-water, made acidic by addition of hydrochloric acid, and extracted with 100 ml of diethyl ether. The ether phase was washed with water, dried over magnesium sulfate and concentrated to give 9.0 g of an oil. The so obtained oil was dissolved in 50 ml of n-hexane and chilled to −20°C to crystallize. There was obtained 4.0 g of 3,7,11,15-tetramethyl-2,4,6,10,14-hexadecapentaenoic acid as pale yellow needles.

In 20 ml of diethyl ether was dissolved 3.0 g of the above-obtained 3,7,11,15-tetramethyl-2,4,6,10,14-hexadecapentaenoic acid. To this solution was added 1 g of triethylamine, and further added 1.1 g of ethyl chlorocarbonate under stirring at room temperature. After 10 minutes, gaseous ammonia was introduced into the solution. The reaction liquid was washed with three 10 ml portions of water, dried over magnesium sulfate, and evaporated to remove the solvent. The residue was purified by almina column chromatography and crystallized from a mixture of acetone and n-hexane (1:2). There was obtained 1.7 g of the desired product as pale yellow crystals.

M.p.: 63—65°C

Analysis for $C_{20}H_{31}NO$

| | C | H | N |
|---|---|---|---|
| Calculated (%) | 79.67 | 10.37 | 4.65 |
| Found (%) | 79.48 | 10.59 | 4.73 |

NMR spectrum (δ, $CDCl_3$): 1.60 (6H, s), 1.68 (3H, s), 1.84 (3H, d, J = 1Hz), 1.9—2.3 (8H), 2.33 (3H, d, J = 1Hz), 5.08 (2H, m), 5.70 (1H, bs), 5.4—6.1 (2H, b), 5.95 (1H, d, J = 11Hz), 6.15 (1H, d, J = 15Hz), 6.82 (1H, dd, J = 15Hz, 11Hz)

Mass spectrum (m/e): 301 ($M^+$)

## Preparation Example 9

*N-(p-Hydroxyphenyl)-3,7,11,15-tetramethyl-2,4,6,10,14-hexadecapentaenoamide*

In 30 ml of tetrahydrofuran was dissolved 3 g of 3,7,11,15-tetramethyl-2,4,6,10,14-hexadecapentaenoic acid. To this solution was added 1 g of triethylamine, and further added 1.1 g of ethyl chlorocarbonate under stirring at room temperature. After 10 minutes, the reaction liquid was poured into 100 ml of water, and extracted with 100 ml of n-hexane. The extract was washed with 50 ml of water and evaporated to remove the solvent. The residue was dissolved in 30 ml of tetrahydrofuran. To this solution was added 1.1 g of p-aminophenol, and the mixture was stirred at room temperature for 30 minutes. To the reaction liquid was added 200 ml of diethyl ether, and the mixture was washed successively with two 50 ml portions of dilute hydrochloric acid and two 50 ml portions of water. The ether phase was dried over magnesium sulfate and evaporated to remove the solvent. The residue was crystallized from ethanol to obtain 3.2 g of the desired product as pale yellow crystals.

M.p.: 163—164°C

Analysis for $C_{26}H_{35}NO_2$

| | C | H | N |
|---|---|---|---|
| Calculated (%) | 79.34 | 8.96 | 3.56 |
| Found (%) | 79.61 | 8.78 | 3.62 |

NMR spectrum (δ, $CDCl_3$): 1.61 (6H, s), 1.68 (3H, s), 1.85 (3H, s), 1.9—2.3 (8H), 2.38 (3H, s), 5.09 (2H, m), 5.76 (1H, bs), 5.96 (1H, d, J = 11Hz), 6.15 (1H, d, J = 15Hz), 6.42 (1H, b), 6.74 (2H, d, J = 8Hz), 6.82 (1H, d, J = 15Hz, 11Hz), 7.22 (1H, bs), 7.32 (2H, d, J = 8Hz)

Mass spectrum (m/e): 393 ($M^+$)

## Preparation Example 10

*N-Ethyl-3,7,11,15-tetramethyl-2,4,6,10,14-hexadecapenta enoamide*

3,7,11,15-Tetramethyl-2,4,6,10,14-hexadecapentaenoic acid and ethylamine were reacted in the same manner as in Preparation Example 9 to obtain the desired product as an oil.

Analysis for $C_{22}H_{35}NO$

| | C | H | N |
|---|---|---|---|
| Calculated (%) | 80.19 | 10.71 | 4.25 |
| Found (%) | 80.44 | 10.79 | 4.38 |

NMR spectrum (δ, $CDCl_3$): 1.15 (3H, t, J = 7Hz), 1.60 (6H, s), 1.67 (3H, s), 1.83 (3H, s), 1.9—2.3 (8H), 2.33 (3H, d, J = 1Hz), 3.27 (2H, qd, J = 7Hz, 6Hz), 5.10 (2H, m), 5.65 (1H, bs), 5.82 (1H, t, J = 6Hz), 5.94 (1H, d, J = 11Hz), 6.10 (1H, d, J = 15Hz), 6.77 (1H, dd, J = 15Hz, 11Hz)

Mass spectrum (m/e): 329 ($M^+$)

## Preparation Example 11

*N,N-Dimethyl-3,7,11,15-tetramethyl-2,4,6,10,14-hexadecapentaenoate*

3,7,11,15-Tetramethyl-2,4,6,10,14-hexadecapentaenoic acid and dimethylamine were reacted in the same manner as in Preparation Example 9 to obtain the desired product as pale yellow crystals.

M.p.: 39—39.5°C

Analysis for $C_{22}H_{35}NO$

| | C | H | N |
|---|---|---|---|
| Calculated (%) | 80.19 | 10.71 | 4.25 |
| Found (%) | 80.26 | 10.83 | 4.32 |

NMR spectrum (δ, $CDCl_3$): 1.60 (6H, s), 1.68 (3H, s), 1.76 (3H, s), 2.09 (3H, d, J = 1Hz), 1.9—2.3

(8H), 3.00 (3H, s), 3.01 (3H, s), 5.10 (2H, m), 5.93 (1H, bs), 5.94 (1H, d, J = 10Hz), 6.18 (1H, d, J = 15Hz), 6.68 (1H, dd, J = 15Hz, 10Hz)

Mass spectrum (m/e): 329 ($M^{+}$)

Example 1

Tablet Preparation

| | |
|---|---|
| N-Ethyl-3,7,11,15-tetramethyl-2,4,6,10,14-hexadecapentaenoamide | 50 g |
| Silicic anhydride | 30 g |
| Crystalline cellulose | 50 g |
| Corn starch | 36 g |
| Hydroxypropylcellulose | 10 g |
| Magnesium stearate | 4 g |

The above-described composition was processed in the conventional manner to produce tablets (180 mg per one tablet).

**Claims**

1. A pharmaceutical composition which comprises a polyprenyl compound having the formula (I):

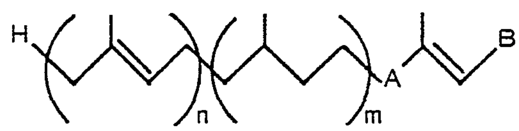

(I)

in which each of *n* and *m* is 0, 1 or 2, n + m is 0, 1 or 2,

A is 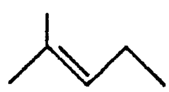 , 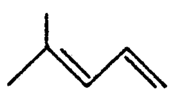 , or 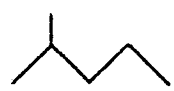 , and B is

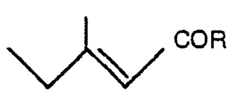 , 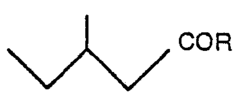 , 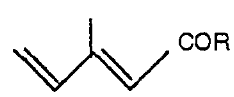 , 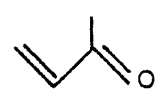 ,

or 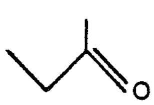 ,

wherein R represents a hydroxy group, a lower alkoxy group, or

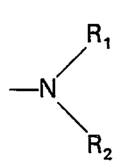 ,

in which each of $R_1$ and $R_2$ is a hydrogen atom, a lower alkyl group or an aryl group, provided that R is a lower alkoxy or

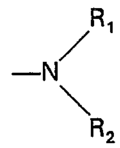 ,

if *n* is 1, *m* is 0, A is

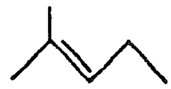 ,

and B is

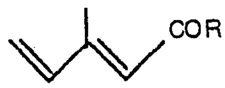 .

2. A pharmaceutical composition according to claim 1 for the prevention and treatment of cancer and skin diseases.

3. A pharmaceutical composition as claimed in claim 2, which comprises 3,7,11,15,19-pentamethyl-2,4,6,10,14,18-eicosahexaenoic acid.

4. A pharmaceutical composition as claimed in claim 2, which comprises 3,7,11,15-tetramethyl-2,4,6-14-hexadecatetraenoic acid.

5. A pharmaceutical composition as claimed in claim 2, which comprises ethyl 3,7,11,15-tetramethyl-2,4,6-14-hexadecatetraenoate.

6. A pharmaceutical composition as claimed in claim 2, which comprises 3,7,11,15-tetramethyl-2,4,6,8,10,14-hexadecahexaenoic acid.

7. A pharmaceutical composition as claimed in claim 2, which comprises 3,7,11,15-tetramethyl-2,4,6,8,10,14-hexadecapentaeno-amide.

8. A pharmaceutical composition as claimed in claim 2, which comprises ethyl 3,7,11,15-tetramethyl-2,4,6,10,14-hexadecapentaenoate.

9. A pharmaceutical composition as claimed in claim 2, which comprises 6,10,14,18-tetramethyl-3,5,9,13,17-nonadecapentaen-2-on.

10. A pharmaceutical composition as claimed in claim 2, which comprises 3,7,11,15-tetramethyl-2,6,10,14-hexadecatetraenoic acid.

11. A pharmaceutical composition as claimed in claim 2, which comprises 6,10,14,18-tetramethyl-5,9,13,17-nonadecatetraen-2-on.

**Patentansprüche**

1. Pharmazeutische Zubereitung, welche eine Polyprenylverbindung der Formel (I) umfaßt:

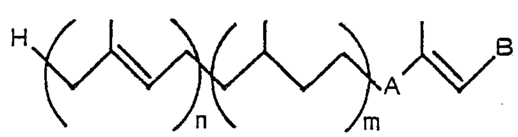 (I)

worin jeweils *n* und *m* die Bedeutung von 0,1 oder 2 haben, n + m = 0, 1 oder 2 ist, A die folgende Bedeutung hat

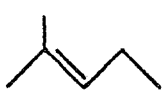 , 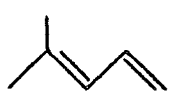 , oder 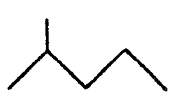 , und B

die folgende Bedeutung hat

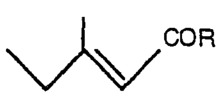 , 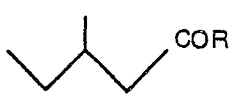 , 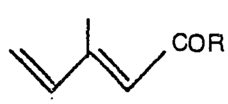 , 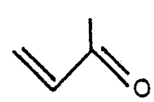 , oder

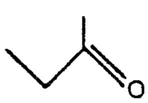 ,

oder

worin R eine Hydroxygruppe, eine Niedrigalkoxygruppe oder

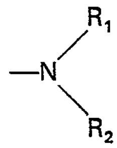

bedeutet, wobei jeweils $R_1$ und $R_2$ ein Wasserstoffatom, eine Niedrigalkylgruppe oder eine Arylgruppe darstellt; vorausgesetzt daß R Niedrigalkoxy oder

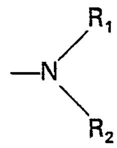

bedeutet, wenn $n = 1$, $m = 0$, A die Bedeutung von

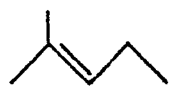 ,

und B die Bedeutung von

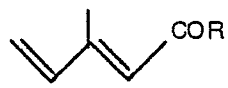

hat.

2. Pharmazeutische Zubereitung gemäß Anspruch 1 zur Prevention und Behandlung von Krebs und Hauterkrankungen.

3. Pharmazeutische Zubereitung gemäß Anspruch 2, dadurch gekennzeichnet, daß diese 3,7,11,15,19-Pentamethyl-2,4,6,10,14,18-eicosahexaensäure darstellt.

4. Pharmazeutische Zubereitung gemäß Anspruch 2, dadurch gekennzeichnet, daß diese 3,7,11,15-Tetramethyl-2,4,6-14-hexadecatetraensäure darstellt.

5. Pharmazeutische Zubereitung gemäß Anspruch 2, dadurch gekennzeichnet, daß diese Ethyl-3,7,11,15-tetramethyl-2,4,6-14-hexadecatetraenoat darstellt.

6. Pharmazeutische Zubereitung gemäß Anspruch 2, dadurch gekennzeichnet, daß diese 3,7,11,15-Tetramethyl-2,4,6,8,10,14-hexadecahexaensäure darstellt.

7. Pharmazeutische Zubereitung gemäß Anspruch 2, dadurch gekennzeichnet, daß diese 3,7,11,15-Tetramethyl-2,4,6,8,10,14-hexadecapentaenoamid darstellt.

8. Pharmazeutische Zubereitung gemäß Anspruch 2, dadurch gekennzeichnet, daß diese Ethyl-3,7,11,15-tetramethyl-2,4,6,10,14-hexadecapentaenoat darstellt.

9. Pharmazeutische Zubereitung gemäß Anspruch 2, dadurch gekennzeichnet, daß diese 6,10,14,18-Tetramethyl-3,5,9,13,17-nonadecapentaen-2-on darstellt.

10. Pharmazeutische Zubereitung gemäß Anspruch 2, dadurch gekennzeichnet, daß diese 3,7,11,15-Tetramethyl-2,6,10,14-hexadecatetraensäure darstellt.

11. Pharmazeutische Zubereitung gemäß Anspruch 2, dadurch gekennzeichnet, daß diese 6,10,14,18-Tetramethyl-5,9,13,17-nonadecatetraen-2-on darstellt.

**Revendications**

1. Composition pharmaceutique qui comprend un dérivé polyprénylique de formule (I)

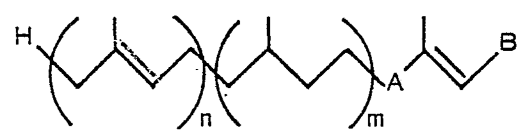 (I)

dans laquelle *n* et *m* ont chacun la valeur 0, 1 ou 2 et n + m a la valeur 0, 1 ou 2, A est un chaînon,

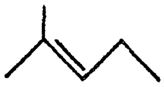 , 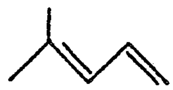 , ou 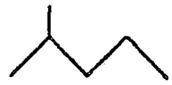

et B est un chaînon 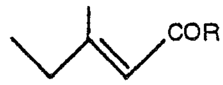 , 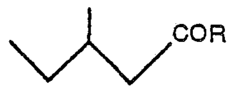 , 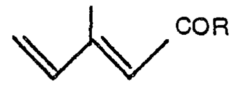 , 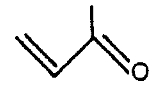

ou 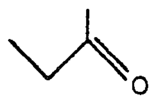 ,

dans lesquels R représente un groupe hydroxy, un groupe alcoxy inférieur, ou un groupe

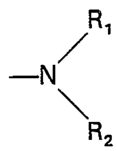

dans lequel $R_1$ et $R_2$ représentent chacun un atome d'hydrogène, un groupe alkyle inférieur ou un groupe aryle; à condition que R soit un groupe alcoxy inférieur ou

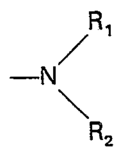 ,

si *n* est 1, *m* est 0,
A est

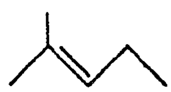 ,

et B est

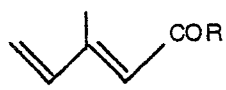

2. Composition pharmaceutique selon la revendication 1 pour la prévention et le traitement du cancer et de maladies de la peau.

3. Composition pharmaceutique telle que revendiqué à la revendication 2, qui comprend l'acide 3,7,11,15,19-pentaméthyl-2,4,6,10,14,18-eicosahexaènoïque.

4. Composition pharmaceutique telle que revendiquée à la revendication 2 qui comprend l'acide 3,7,11,15-tétraméthyl-2,4,6,14-hexadécatétraènoïque.

5. Composition pharmaceutique telle que revendiqué à la revendication 2 qui comprend le 3,7,11,15-tétraméthyl-2,4,6,14-hexadécatétraènoate d'éthyle.

6. Composition pharmaceutique telle que revendiquée à la revendication 2, qui comprend l'acide 3,7,11,15-tétraméthyl-2,4,6,8,10,14-hexadécahexaènoïque.

7. Composition pharmaceutique telle que revendiquée à la revendication 2, qui comprend le 3,7,11,15-tétraméthyl-2,4,6,8,10,14-hexadécapentaènoamide.

8. Composition pharmaceutique telle que revendiquée à la revendication 2, qui comprend le 3,7,11,15-tétraméthyl-2,4,6,10,14-hexadécapentaènoate d'éthyle.

9. Composition pharmaceutique telle que revendiquée à la revendication 2, qui comprend la 6,10,14,18-tétraméthyl-3,5,9,13,17-nonadécapentaène-2-one.

10. Composition pharmaceutique telle que revendiquée à la revendication 2, qui comprend l'acide 3,7,11,15-tétraméthyl-2,6,10,14-hexadécatétraènoïque.

11. Composition pharmaceutique telle que revendiquée à la revendication 2, qui comprend la 6,10,14,18-tétraméthyl-5,9,13,17-nonadécatétraène-2-one.